# EUROPEAN PATENT SPECIFICATION

(11) **EP 2 340 123 B1**
(45) Date of publication and mention of the grant of the patent: **26.06.2019**
(21) Application number: 08801905.4
(22) Date of filing: 03.09.2008
(51) Int. Cl.: B01L 3/14, G01N 33/49, G01N 33/50, G01N 33/537, G01N 33/577, G01N 33/80

(54) **REACTION VESSEL CAPABLE OF DETECTING AGGLUTINATED ANTIBODY LOADED ERYTHROCYTES, METHOD FOR DETECTING AGGLUTINATED ANTIBODY LOADED ERYTHROCYTES, AND KIT OF PARTS**
AGGLUTINIERTE ANTIKÖRPER GELADENE ERYTHROZYTE ERFASSENDES REAKTIONSGEFÄSS, VERFAHREN ZUR ERFASSUNG VON AGGLUTINIERTEN ANTIKÖRPER GELADENEN ERYTHROZYTEN UND TEILESATZ
CUVE DE RÉACTION APTE À DÉTECTER DES AGGLUTINATS ÉRYTHROCYTAIRES CHARGÉES D'ANTICORPS, PROCÉDÉ POUR LA DÉTECTION DES AGGLUTINATS ÉRYTHROCYTAIRES CHARGÉES D'ANTICORPS ET COFFRET D'ÉLÉMENTS

(43) Date of publication of application: 06.07.2011
(73) Proprietor: Sanquin Reagents B.V., 1066 CX Amsterdam (NL)
(72) Inventor: VAN DER DONK, Eric, Marinus, Maria, NL-1181 BD Amstelveen (NL); SPRENGERS, Ferry, Gerardus, Cornelis, NL-1319 CW Almere (NL); VAN EIJK, Ronald, Victor, Wilhelmus, NL-3708 GC Zeist (NL); MELSERT, Roeland, NL-1851 ZS Heiloo (NL)
(74) Representative: van Kooij, Adriaan
(86) International application number: PCT/EP2008/007325
(87) International publication number: WO 2010/025756

(56) References cited:
- EP-A- 0 849 595
- WO-A-95/30904
- WO-A-98/16832
- WO-A-99/50673
- US-A1- 2007 231 834

## Description

The present invention relates to a micro column capable of detecting carrier-bound analyte complexes, and, more particularly, erythrocyte-bound antibody complexes or agglutinates. Further, the present invention relates to a method for detecting carrier-bound analyte complexes, and, particularly, erythrocyte-bound antibody complexes, comprising the use of the present micro column. Furthermore, the present invention relates to a kit of parts comprising the present micro column for use in the present method. The present micro column, method, and kit of parts are preferably used in the field of blood group serology, and, especially, blood group assays and antibody detection.

In a clinical setting, a major objective of blood group serology, and, especially, blood group assays and antibody detection, is to obtain compatible red blood cell or erythrocyte preparations from a donor for blood transfusion of an acceptor. For this, assays are routinely performed such as blood group typing, antibody screening and identification, and (blood) compatibility assays by performing cross matches.

In general, the majority of these assays are based on the principle of agglutination, i.e., the formation of carrier-bound analyte complexes, such as erythrocyte-bound antibodies complexes. Such complexes, clearly microscopically visible or, in some cases, already visible by the naked eye, can comprise from about 50 up to thousands of carrier-bound analytes, such as erythrocyte-bound antibodies.

In a mammal, and particularly a human mammal, specific antigenic determinants on the cell membrane of red blood cells, or erythrocytes, are characterizing the so-called blood group of said mammal. The information for expressing blood group antigens is generally genetically determined at the genomic level.

A well known, and generally used, blood group system is the so-called A, B, AB, and O system (AB0-system), discovered in 1900 by Karl Landsteiner. Different blood groups in this system are designated A, B, AB, and O.

In addition to the ABO-system, more than 400 red blood cell blood groups are known, the majority of these being clustered in blood group systems with diverging clinical significance. Specific antibodies to these other blood group systems can be formed after immunization with the corresponding blood group antigen, for example during blood transfusion or pregnancy, and may cause problems during a blood transfusion or pregnancy thereafter.

Presently, for transfusion practices, the ABO-system is the most important blood group system. This because every individual, characterized by a specific blood group, has antibodies in his serum against the blood group(s) that are not present. For example, an individual characterized as blood group A, will have anti-B antibodies in his serum, and vice versa.

These antibodies are so-called "naturally occurring" antibodies and are in general strong IgM type antibodies. The IgM antibodies are capable of causing a direct, i.e., without a bridging reagent, such as an anti-IgM antibody, complex formation or agglutination of, for example, erythrocytes exposing a blood group antigen against which the antibody is directed.

Blood transfusion reactions in an individual (recipient) induced by allo-antibodies, raised against foreign erythrocytes or red blood cells are called hemolytic transfusion reactions. This because they are generally accompanied by a strongly accelerated, and often deadly, breakdown of erythrocytes. Therefore, it of major importance to prevent hemolytic transfusion reactions by careful serological examination before a blood transfusion is performed. For this, several types of tests are generally carried out.

In general, both donor and recipient are typed for the AB0-blood group system and Rhesus D antigen. These must be identical, or compatible, for both the donor and recipient. The AB0-blood group found on the red blood cells can, for example, be confirmed by performing a so-called reverse AB0 typing test on the antibodies present in the serum.

Next, a screening of the serum of the recipient is performed for the presence of red blood cell antibodies directed against all other blood groups beside the AB0-system. If an antibody is found, it must be identified in order to select donor blood which is negative for the corresponding blood group antigen.

Finally, a cross-match between donor red blood cells and recipient serum can be performed to find out whether the donor and the recipient are in deed compatible.

In general, blood group antibodies are immunoglobulins of the IgG or IgM type. The antigen-antibody reaction, such as the present carrier-analyte reaction forming carrier-bound analytes, is dependent, amongst others, on ionic binding, hydrogen bridges and hydrophobic effects (displacement of water). The strength of the binding between the binding pocket of an antibody and an epitope is designated as "affinity".

Antibodies which are capable of agglutinating red blood cells under all conditions are designated agglutinins or complete antibodies (in general IgM antibodies). Antibodies which bind to (sensitize) red blood cells, but cause no direct agglutination, are called incomplete antibodies (in general IgG antibodies).

Red blood cell antigens and their corresponding antibodies are often detected by means of agglutination reactions, which can take place in a physiological salt solution. In practice, agglutination tests can be rendered more sensitive by using, for example, a medium having a low ionic strength, proteolytic enzymes (e.g. bromelin, papain or ficin), polycations (e.g. polybrene), macromolecules (e.g. albumin), or polymers (e.g. polyethylene glycol (PEG) or dextrane). A large number of serological tests is known.

Presently, the most common, and generally used, serological tests are the tube method, micro column tests, and tests in micro plates. These techniques can be further divided into techniques based on agglutination, i.e. complex formation, and techniques based on a solid-phase (affinity) principle.

Although serological test based on DNA techniques are available, for example, blood group typing, their application in the field of serology is still limited. In addition, techniques based on fluorescent labels or magnetic beads are available.

When considering the present major clinical applications in the field of serology, the tube method, micro column tests, and tests in micro plates tests are widely used and will be further detailed below.

The tube method is a widely used test allowing prolonged incubations with antibodies. Red blood cells, after a reaction with antibodies, can be sedimented, or centrifuged, to accelerate the agglutination reaction.

An important, and widely applied, variant of the tube method test is the antiglobulin test or Coombs test, described by Moreschi in 1908 (Zbl. Bakt. 46: 49, 1908) and reintroduced in 1945 by Coombs et al. (Lancet 2: 15, 1945 and Brit. J. Exp. Path. 26: 255, 1945). The Coombs test is based on the principle that red blood cells loaded with, for example, incomplete antibodies of the IgG type can be agglutinated through the addition of antiglobulin serum. In the test, three phases can be distinguished.

The first phase is the sensitization phase. During this phase, antibodies bind to the corresponding antigen structures on the red blood cells (sensitization of red blood cells). When binding, thereby forming carrier (erythrocyte)-bound analytes (antibodies), has occurred, a second phase is initiated, also designated as the wash phase. In this wash phase, substantially all non-bound antibodies are removed from the incubation mixture.

The third phase is designated as the antiglobulin phase, in which antiglobulin serum is added to the washed sensitized, i.e., antibody loaded, cells. This causes binding of sensitized cells to each other resulting in the formation of complexes or agglutinates comprised of clustered, i.e., from about 50 to thousands, red blood cells.

When performing the Coombs test, it is necessary, before adding the antiglobulin serum, to wash very thoroughly and frequently, and thus this step is very time consuming. Insufficient removal of non-bound antibodies can lead to inactivation of the antiglobulin serum. Other disadvantages of the test are the need for promptly reading the results by a trained professional, that the test results cannot be preserved, the test is less reproducible because of manual reading of the test results, and difficulties in automating the test.

As indicated, the washing step in the Coombs test is very time consuming. Graham et al. (Transfusion 22: 408, 1982 and P.L. Mollison, Blood transfusion in clinical medicine, Blackwell, Oxford, p. 512, 1983) developed a technology rendering the wash step redundant. This technology was commercialized by Ortho Diagnostic Systems Inc. in a test system marketed as Simwash®.

In this system, red blood cells are separated from the serum by means of a centrifugation step. The separation is based on the observation that the specific gravity of serum is lower than that of red cells. If a mixture of cells and serum is applied on top of a layer of medium with a density between the density of the cells and that of the serum, the (sensitized) red blood cells will be separated in a sedimentation or centrifugation step from the serum (containing the non-bound antibodies). Thus, the red blood cells are sedimentated or centrifuged out of the incubation mixture. In this way, a triple or quadruple washing step, needed in the Coombs test, is reduced to a single centrifugation step. Thereafter, the sensitized cells are incubated with antiglobulin serum and the formation of complexes or agglutinates is detected.

A further improvement of the test is provided by centrifuging red blood cells through a column of transparent, inert, solid particles (micro column test) such as a Sephadex® gel or glass beads (Dalton et al., 1970, (Beckton Dickinson & Co., U.S. Pat. No. 3,492,396). Because of their size, in theory, agglutinates are not capable to pass through the column in contrast to the non-agglutinated (individual) cells. As a consequence, agglutinates will be retained on top, in the upper part, or in the remainder, of the column depending on the column material used and/or the size of the agglutinates. This in contrast with non-agglutinated cells that readily pass through the column.

The principle of using a column of inert and solid particles for retaining agglutinates was further refined by LaPierre et al. (Transfusion 30: 109-113, 1990 and European Patents 0 194 212 and 0 305 337). This system combines the principles of Simwash® and the use of solid inert particles to discriminate between complexes or agglutinates and non-agglutinates.

In this test system, use is made of small columns filled with Sephadex® gel. Use can be made of columns comprising antibodies (e.g. for blood group typing) or no antibodies (e.g. for reverse ABO typing).

In order to perform a Coombs test, use is made of gel columns containing antiglobulin serum. After incubation, the gel columns are centrifuged. In case of a negative reaction, i.e., no carrier-bound analytes complexes are formed, all (individual) red blood cells will end up at the bottom of the micro column; if the test is positive, the red blood cell complexes, or agglutinates, will be more or less retained by the column, i.e., will, after centrifugation, be visible on top or somewhere in the column. In case of weak reactions, red blood cells will sediment partly resulting in red blood cells in the column and at the bottom of the column. This test is marketed by DiaMed AG (DiaMed-ID Microtyping System), Grifols (DianaGel, DG Gel® cards) and Bio-Rad/Sanofi (ScanGel® cards) and has been used in the past by Diagast Laboratoires (Chromatest).

A comparable micro column system has been described using non-compressible micro particles instead of gel material as inert material to retain the complexes or agglutinates formed (European Patents 0 485 228, 0 725 276, 0 755 719 and US patents 5,552,064 and 5,650,068). This test is marketed by Ortho (Biovue System), using glass beads as non-compressible micro particles.

A number of drawbacks are associated with the above micro column systems. For example, a special centrifuge is required for correct performance of the test. Also, special reading equipment is required for automatic reading of the test as well as special equipment for automation of the entire test.

A general disadvantage of the above micro column agglutination tests is the occurrence of "smear formation", especially when weak reactions are tested, resulting in uncertainty when reading and interpreting the strength of the reaction results, both at reading by the naked eye and at automated reading of the results. A red smear of red blood cells (mostly small red blood cell clusters comprised of 2 to less than 50 cells, also designated as rosettes) can be seen over the entire micro column or part thereof. These smears are, for example, caused when:
1. small red blood cell clusters or rosettes, formed during incubation when testing weak IgM antibodies, sediment partly since they penetrate deeper into the gel of the micro column than large and strong agglutinates;
2. small clusters or rosettes are formed in the gel itself due to (optionally) present antiglobulin serum when testing weak IgG antibodies;
3. weak agglutinates already formed are broken down into small clusters or rosettes by "shear forces" during a centrifugation step caused by a centrifugal force exerted on the agglutinates and the hindering force exerted on the agglutinates by the presence of the gel particles (Philips et al., Transfusion Medicine 7: 47-53, 1997).

Another general drawback of the above micro column agglutination tests is that problems often occur when demonstrating weak agglutinates, for instance in the case of weak AB0-incompatibilities or in the case of some other weak IgM antibodies reactive at 37 °C, like anti-P1 and anti-Lea (Cummins & Downham, Lancet 343: 1649, 1994; Philips et al., Transfusion Medicine 7: 47-53, 1997; Cheng et al., Clin. Lab. Haem. 17: 81-84, 1995).

The shear forces during centrifugation can cause weak agglutinates to disintegrate into several small red blood cell clusters or rosettes (or, in extreme cases, into individual (sensitized) red cells), that will not be detected since they are too small to be sieved by the gel particles and therefore will sediment, resulting in an increase of the number of false negative reactions.

Another approach in serology testing is based on the solid-phase (affinity) principle as an alternative for direct (complex formation or agglutination without a bridging reagent) and indirect (complex formation or agglutination with a bridging reagent) reactions for blood group typing, antibody screening, antibody identification and cross match. Applications and advantages of the use of affinity solid-phase techniques in serology have been described by Rosenfield (Abstracts, 15th Cong. Int. Soc. Blood Trans., Paris, pp. 27-33, 1976 and US Patent 4,275,053). Here, amongst others, red blood cells were used which had been coupled to the surface of plastic tubes.

More recently, systems have been described by Plapp et al. (Am. J. Clin. Path. 82: 719-721, 1984), Bayer et al. (US Patent 4,608,246), Rachel et al. (Transfusion 25: 24-26, 1985), Plapp et al. (The Lancet 1465-1466, 1986) and Uthemann et al. (US Patent 4,925,786, European Patent 0 363 510 and Transfusion 30: 114-116, 1990).

Microplates in combination with the solid-phase principle are used by, among others, Biotest AG (Erytype for typing of red cell antigens and Solidscreen II for antibody diagnostics), Immucor Inc. (Capture-R systems for antibody screening and identification) and Sanquin (Microtype for typing of red cell antigens) and have further been described by Llopis et al. (Vox Sanguinis 70: 152-156, 1996 and Vox Sanguinis 72: 26-30, 1997), Ohgama et al. (Transfusion Medicine 6: 351-359, 1996), Chateau et al. (La Gazette de la Transfusion 131: 38-41, 1997 and French Patent 94 05123) and Den Boer et al. (US Patent 6,303,390).

The solid-phase affinity tests are not limited to microplates. Pernell (Sanofi Pasteur/BioRad, European Patent 0 594 506) describes an affinity gel test in which an immunoglobulin-binding substance (such as protein A, protein G or anti-human IgG) is immobilized on the gel matrix. Incubation of red blood cells and antibodies occurs above the gel matrix. After this incubation phase, centrifugation of the gel column takes place. If antibodies are bound to the red blood cells, these sensitized red cells will bind to the immunoglobulin-binding gel matrix and, hence, will bind to the upper part of the gel column. Red blood cells to which no antibodies are bound will not be bound and end up at the bottom of the column.

This principle of binding of sensitized cells to a solid phase has previously been described by GE Healthcare / Amersham/Pharmacia (Cell Affinity Chromatography, Uppsala, Sweden, 1984). Therein, Pharmacia describes the purification of red blood cells based on the presence or absence of certain blood group antigens using protein A-Sepharose 6MB. Red blood cells with the A antigen on the surface (blood group A red blood cells) to which anti-A antibodies are bound, can be separated by binding to said gel material (via protein A) from red blood cells that do no possess the A antigen.

Therefore, the test described by Sanofi Pasteur is a combination of this principle and the centrifugation step for separating red blood cells, whether sensitized or not, and non-bound antibodies as used, for example, in the micro column system of DiaMed.

A comparable system has also been described by Gamma/Immucor (WO 95/31731 and WO 98/16831 and US Patents 5,665,558 and 5,905,028) using micro tubes containing two layers of immunoreactive gel particles, a first layer having protein G coupled to the surface of the particles and a second layer having protein A coupled to the particles.

Two types of assays can be carried out: 1. a direct assay, where antibodies specific for blood group antigens to be tested are coupled to the protein G and a sample of red cells being added to the reaction tube above the gel; 2. an indirect assay, where samples of red blood cells, serum or plasma are incubated with a known antibody or antigen reagent in the reaction tube above the gel. In both assay types the reaction tube is subsequently centrifuged to force to the bottom of the reaction tube red blood cells that do not attach to the immunoreactive gel particles.

An alternative to these tests, thereby improving the results, has been described by Van der Donk et al. (Dutch Patent 1008738 and European Patent 1 064 556). Apart from an optionally present affinity ligand for binding of red cells sensitized with IgG-antibodies, here two affinity ligands are immobilized on a gel matrix in micro columns, being an affinity ligand for binding of red blood cells sensitized with IgM-antibodies and an affinity ligand for binding of red blood cells loaded with complement components, caused by complement dependent antibodies. This causes an (unexpected) improved sensitivity for detection of weak reactions mediated by IgM-antibodies reactive at 37°C, for example: 1. when detecting weak AB0 incompatibilities, or IgM type anti-Le or anti-P1 antibodies; 2. when typing weak expressions of blood group antigens or variants thereof, using typing reagents containing monoclonal IgM antibodies. An improved sensitivity is also observed for detection of complement dependent (IgG) antibodies, some of which have been described to be detectable only by means of anti-complement activity (Mollison et al., Blood Transfusion in Clinical Medicine, Blackwell Scientific Publications, 1992) .

A major disadvantage of the above described affinity gel test systems is that a relatively high amount of costly ligand molecules is necessary, while only a fraction thereof is effectively utilized.

Since use is being made of gel particles originally meant for chromatographic applications, immobilization of ligands on these gel particles occurs not only on the outer surface of the gel particles, where they can interact with the red blood cells, but also on the relatively large inner surface of the gel particles, where the red blood cells cannot penetrate.

Another disadvantage is that a-specific interactions can occur between (non sensitized) red blood cells and the gel matrix and/or the immobilized ligands, resulting in apparent binding of red blood cells to the gel matrix and thereby in an increase in the amount of false positive reactions.

Considering the above, it is an object of the present invention, amongst others, to obviate at least part of the above disadvantages associated with the known serology test systems, and especially the serology test systems based on (micro) columns.

According to the present invention, this object is met by the micro column as defined in the appended claims.

Especially, this object is met by providing a micro column, capable of detecting carrier-bound-analyte complexes, comprising at least three fluid communicating elongated compartments, wherein said micro column comprises:
a) a first elongated compartment (3) capable of receiving erythrocytes and/or antibodies, thereby providing a sample with a first liquid density, for forming antibody-loaded-erythrocytes, but not agglutinated-antibody-loaded-erythrocytes, wherein said first compartment (3) is in liquid communication with;
b) a second elongated compartment (2) of about 17 µl consisting of a medium with a second liquid density, wherein said second liquid density is higher than said first liquid density, wherein said medium comprises at least one compound selected from the group consisting of anti-IgG antibodies, anti-IgM antibodies, anti-C3d antibodies, anti-IgA antibodies, anti-C3c antibodies, and functional fragments thereof and wherein said second liquid density is between 1.01 to 1.09 g/ml, preferably between 1.02 and 1.06 g/ml, more preferably between 1.02 and 1.04 g/ml, for forming agglutinated-antibody-loaded-erythrocytes, and wherein said second compartment (2) is in liquid communication with;
c) a third elongated compartment (1) of about 5 µl consisting of inert beads, comprised in the medium according to (b), capable of providing a barrier for said agglutinated-antibody-loaded erythrocytes and not for said antibody-loaded-erythrocytes, erythrocytes and/or antibodies;
wherein the ratio of the length of said elongated second compartment (2) to said third elongated compartment (1) is between 1.1 to 1.7 thereby providing complex formation and agglutination of said antibody-loaded-erythrocytes at the interface of said second (2) and third (1) elongated compartment.

Within the context of the present invention, carrier-bound analyte complexes and, particularly, agglutinated antibody loaded erythrocytes are defined as complexes comprising at least 50 carrier-bound analytes and agglutinated antibody loaded erythrocytes, respectively.

In the first compartment, incubation of the carrier, such as red blood cells, and analyte, such as antibodies, results in the formation of carrier-bound analytes, such as antibody sensitized red blood cells. It is of importance to note that substantially no complex formation, i.e., agglutination, occurs in the first compartment.

However, this inherently implies that, in the case of IgM antibodies, the spontaneous formation of complexes or agglutinates in the first compartment must be substantially prevented. The skilled person, by following the Heidelberger curve, is readily capable to choose the concentrations of carrier and analyte in such a way that complex formation or agglutination is substantially prevented in the first compartment, for example, by using a high concentration of red blood cell antibodies in combination with a low concentration of red blood cell antigens.

It is noted that in case of IgG antibodies, no additional measures are needed to prevent complex formation or agglutination. This because IgG sensitized erythrocytes are inherently substantially not capable of forming complexes or agglutinates without the further addition of a bridging reagent, such as an anti-IgG antibody.

After the incubation phase in the first compartment, a centrifugation takes place, such that the carrier-bound analytes are centrifuged through the second compartment, while the analyte remains above the present second compartment. In this manner, the analyte, such as antibodies, present in the sample, are capable of binding the carriers, such as red blood cells, without being hindered by a premature contact with free antibodies present in the second compartment. Vice versa, the optional free antibodies present in the second compartment cannot be inactivated by a premature contact with the analyte, such as an antibody containing sample.

Further, because of the density of the medium in the second compartment, the unbound analyte will be effectively separated from the carrier.

Red blood cells sensitized with analyte, such as complement factors and/or IgG antibodies and/or IgM antibodies and/or IgA antibodies, are allowed to react with the optional free antibodies present in the second compartment, thereby reinforcing sensitized red blood cells into a loosely associated 3-dimensional network of red blood cells, followed by subsequent formation of complexes or agglutinates of sensitized red blood cells upon landing on the top of the third compartment.

In order to obtain this effect, it of critical importance that the second compartment has a sufficient length in relation to the third compartment in combination with a sufficient density of the medium in the second compartment.

The present inventors have surprisingly found that when the fluid density is between 1.01 to 1.09 g/ml and the ratio of the length of the elongated second compartment to the third elongated compartment is between 1.1 to 1.7, complex formation and agglutination is provided at the interface of the compartments, thereby providing the observed increased sensitivity of the present micro column over the prior art systems.

Further, since complex formation or agglutination takes only place at the interface of the second and third compartment, shear forces occurring during passage of agglutinates through the column matrix in other column agglutination test systems will not occur according to the present invention because complexes or agglutinates do not pass through the column matrix.

Using the micro column according to the present invention, it was found possible to demonstrate, in addition to IgG antibodies, complement-dependent antibodies with a high sensitivity.

In addition, it also was possible to demonstrate IgM antibodies, including weak IgM antibodies, reactive at 37 °C, with a high sensitivity.

Further, the reading of the test results was improved and more univocal because of detection of red blood cells at two levels, at the bottom (negative reaction) and at the interface of the third compartment (positive reaction).

According to a preferred embodiment of the present micro column, the second fluid density is between 1.02 and 1.06 g/ml, more preferably between 1.02 and 1.04 g/ml.

According to another preferred embodiment, the ratio of the length of the elongated second compartment to the third elongated compartment is between 1.3 to 1.5.

According to yet another preferred embodiment of the present micro column, the top-end diameter of the first elongated compartment is larger than the bottom-end diameter of the first elongated compartment for facilitating sample receiving, i.e., carrier and analyte.

Not according to the presently claimed micro column, the diameter of the second and third elongated compartment is substantially equal to the diameter of the bottom-end diameter of the first elongated compartment.

Preferably, the present medium defining the second elongated compartment comprises 2% to 4% of dextran and 1% to 3% serum albumin with a pH of at least 6, and, more preferably, further comprises a physiological salt solution, a chelator and a mono- or disaccharide.

The presence of macromolecules and polymers in the present medium defining the second compartment increases the interaction between the red blood cell antigens and antibodies and between (optionally) free anti-human antibodies and red blood cell antibodies present on the red blood cells. In this way association of sensitized red blood cells is promoted and, subsequently, complex formation or agglutination of red blood cells takes place upon sedimentation on top of the gel matrix in stead of inside the gel matrix (in the case of IgG-type antibodies and/or weak IgM-type antibodies).

In a more preferred embodiment, the present medium defining the second elongated compartment further comprises 15 to 25 mM EDTA, 60 to 80 mM NaCl, 20 to 40 mM glucose in a phosphate buffered solution (PBS).

In a most preferred embodiment, the present medium, defining the second elongated compartment, comprises 2.5% dextran, 2% serum albumin, 20 mM EDTA, 70 mM NaCl, and 30 mM glucose in a phosphate buffered solution (PBS).

According to the invention, the above medium, defining the second elongated compartment, further comprises at least one compound selected from the group consisting of anti-IgG antibodies, anti-IgM antibodies, anti-C3d antibodies, anti-IgA antibodies, and anti-C3c antibodies, more preferably anti-IgG antibodies, anti-IgM antibodies, and anti-C3d antibodies.

The above addition of antibodies strongly increases the sensitivity, and especially the sensitivity for weak IgM-type antibodies (reactive at 37°C), complement-dependent antibodies, and normal IgG-type antibodies. The presence of anti-human IgG antibodies, anti-human IgM antibodies and anti-human complement antibodies in the medium defining the second elongated compartment reinforces the network of sensitized red blood cells before the actual complex forming or agglutination reaction takes place.

According to a preferred embodiment, the inert beads, comprised in the medium as defined above and defining the third elongated compartment, have an average diameter of between 10 to 150 µm, preferably between 20 to 50 µm.

Further, according to a preferred embodiment, the inert beads, comprised in the medium as defined above and defining the third elongated compartment, are selected from the group consisting of glass, sepharose, acryl, crosslinked agarose, and sephadex beads.

According to the present invention, the carrier and analyte are erythrocytes and antibody, respectively.

According to the present invention, the carrier-bound analytes and the carrier-bound analyte complexes are antibody loaded erythrocytes and agglutinated antibody loaded erythrocytes, respectively.

The present micro column as defined above provides many advantages over the serology test systems of the prior art, especially with respect to an increased sensitivity and the absence of "smear" formation in the column compartment, not only allowing an increased sensitivity, but also reducing the number of false negative results, and a high automation potential of the test.

Considering the above advantageous properties of the present micro column, the present invention also relates to a method for detecting agglutinated antibody-loaded-erythrocytes as defined in claim 10.

According to a preferred embodiment of the present method, the incubation comprises 1 second to 30 minutes at 15 to 37 °C.

The centrifugation according to the present method preferably comprises 1 to 3 minutes at 50 to 100xg; 0.5 to 1.5 minutes at 150 to 250xg; and 5-10 minutes at 1500 to 2000xg, more preferably, 2 minutes at 75xg; 1 minute at 200xg; and 7 minutes at 1790xg.

According to the present method the analyte is antibody; the carrier is erythrocytes; the carrier-bound analyte is antibody loaded erythrocytes; and the carrier-bound analyte complex is agglutinated antibody loaded erythrocytes.

The present invention also relates to a kit of parts for carrying out the present method comprising:
a) at least one micro column as defined above;
b) instructions for use; and
c) optionally, one or more control analytes and/ or carriers.

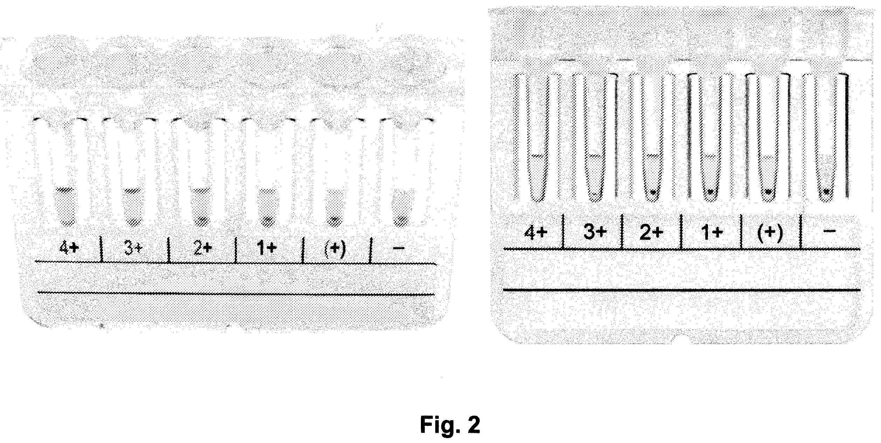

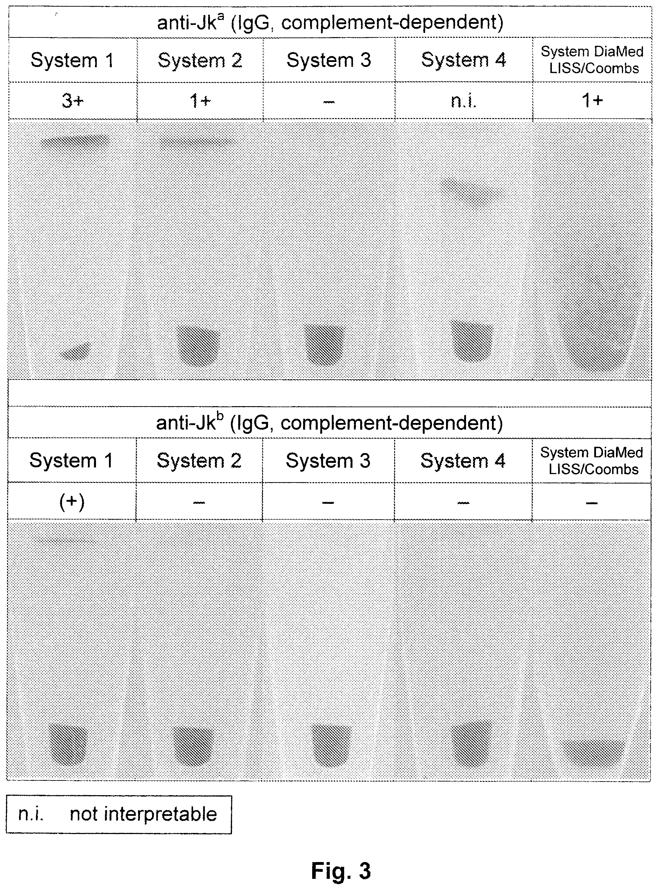

The present invention will further be detailed in the following examples comprising, and describing, preferred embodiments of the present invention. In the examples, reference is made to the appended figures, wherein:
- **Figure 1:**: shows a test card with six micro columns. In a micro column (7) closed at the bottom, a gel matrix of inert particles (1) is provided. On this gel matrix there is a layer of separation medium of high density (2). The incubation compartment (3) is formed by a widening at the top of the micro column. A micro column test system can be integrated into a base plate (6) and be placed in a centrifuge using supporting and guiding elements (9, 10, 11).
The reference numerals denote:
1 Matrix of inert particles
2 Separation medium of high density
3 Incubation compartment
4 Micro column wall
5 Micro column bottom
6 Base plate
7 Micro column
8 Recess
9 Top surface
10 Slot
11 Support
- **Figure 2:**: shows the various possible reaction strengths. The strength indications denote:
4+ all agglutinated cells on top of the inert gel matrix, no cells at the bottom
3+ more agglutinated cells on top of the inert gel matrix than non-agglutinated cells at the bottom
2+ as many agglutinated cells on top of the inert gel matrix as non-agglutinated cells at the bottom
1+ fewer agglutinated cells on top of the inert gel matrix than non-agglutinated cells at the bottom
(+) very few agglutinated cells on top of the inert gel matrix, many non-agglutinated cells at the bottom
- no agglutinated cells on top of the inert gel matrix, all cells at the bottom
- **Figure 3:**: Test results of 5 micro column systems: System 1: The test system as described having free anti-human IgG, anti-human IgM and anti-human C3d in the high density medium; System 2: As system 1, but with free anti-human IgG alone in the high density medium; System 3: As system 1, but without free antibodies in the high density medium; System 4: As system 1, but without the layer of high density medium above of the gel matrix; System DiaMed LISS/Coombs: DiaMed ID LISS/Coombs micro column test card, containing free anti-human IgG and anti-human C3d in the gel matrix.

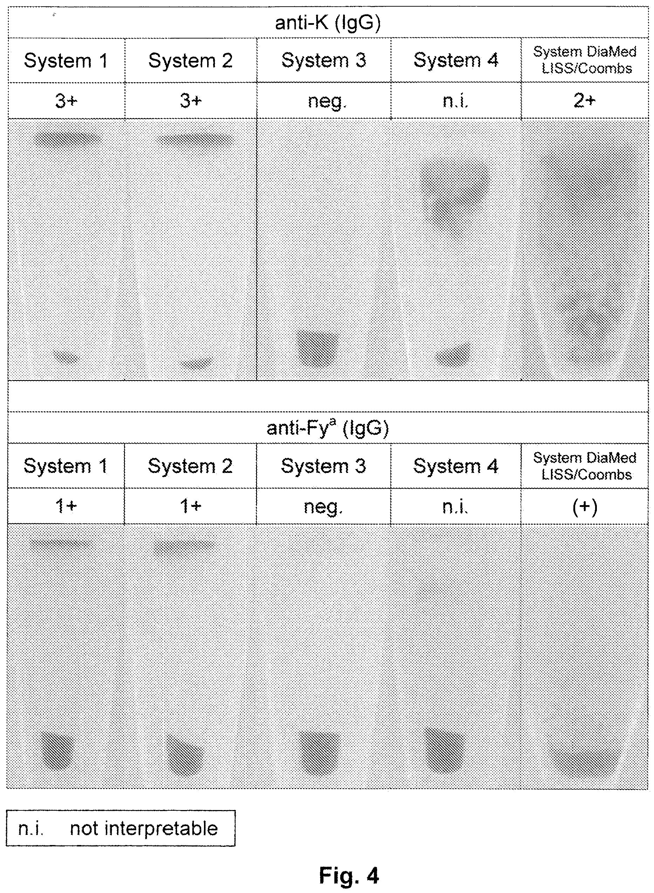

- **Figure 4:**: Test results of 5 micro column systems: System 1: The test system as described having free anti-human IgG, anti-human IgM and anti-human C3d in the high density medium; System 2: As system 1, but with free anti-human IgG alone in the high density medium; System 3: As system 1, but without free antibodies in the high density medium; System 4: As system 1, but without the layer of high density medium above of the gel matrix; System DiaMed LISS/Coombs: DiaMed ID LISS/Coombs micro column test card, containing free anti-human IgG and anti-human C3d in the gel matrix.

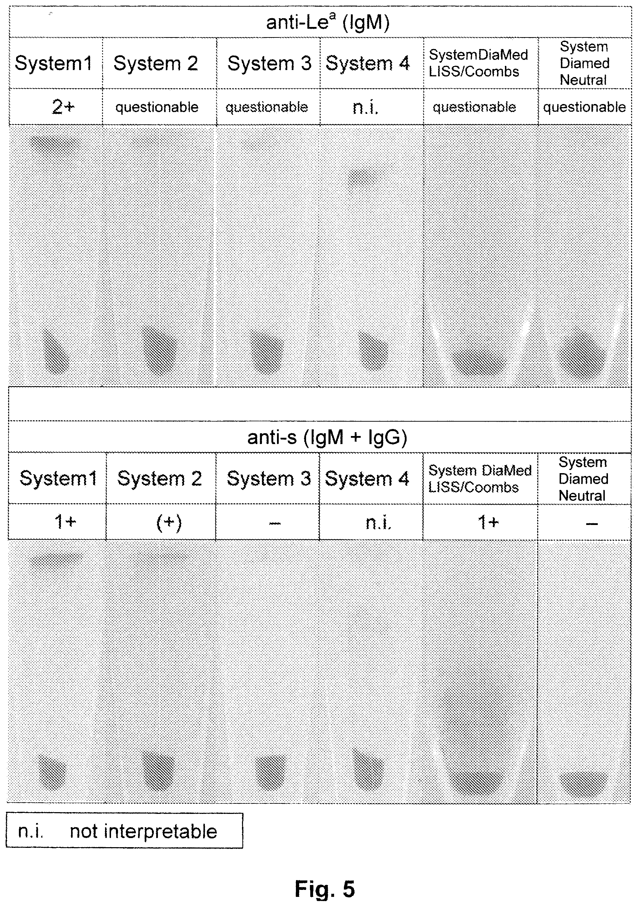

- **Figure 5:**: Test results of 6 micro column systems: System 1: The test system as described having free anti-human IgG, anti-human IgM and anti-human C3d in the high density medium; System 2: As system 1, but with free anti-human IgG alone in the high density medium; System 3: As system 1, but without free antibodies in the high density medium; System 4: As system 1, but without the layer of high density medium above of the gel matrix; System DiaMed LISS/Coombs: DiaMed ID LISS/Coombs micro column test card, containing free anti-human IgG and anti-human C3d in the gel matrix; System DiaMed neutral: DiaMed ID NaCl, enzyme test and cold agglutinins micro column test card, containing no free antibodies in the gel matrix.

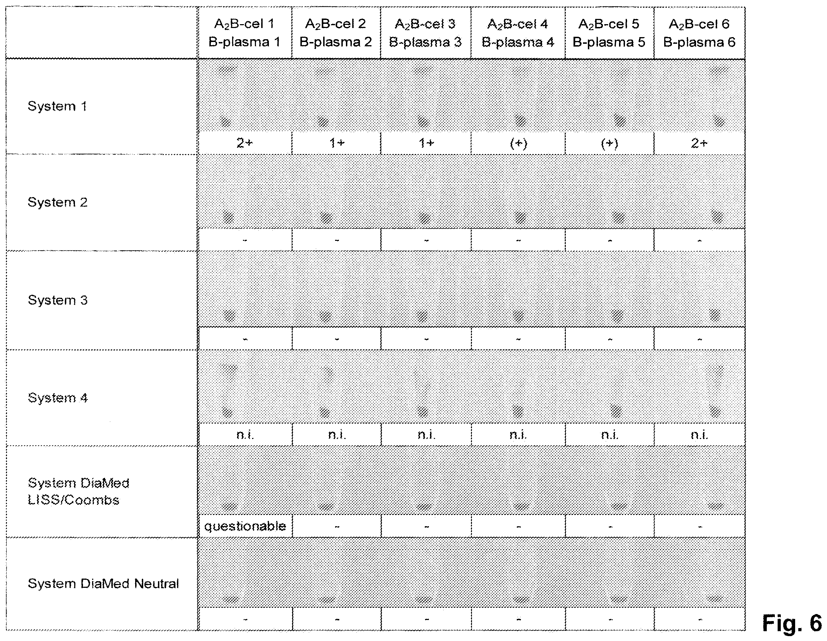

- **Figure 6:**: Test results of 6 micro column systems: System 1: The test system as described having free anti-human IgG, anti-human IgM and anti-human C3d in the high density medium; System 2: As system 1, but with free anti-human IgG alone in the high density medium; System 3: As system 1, but without free antibodies in the high density medium; System 4: As system 1, but without the layer of high density medium above of the gel matrix; System DiaMed LISS/Coombs: DiaMed ID LISS/Coombs micro column test card, containing free anti-human IgG and anti-human C3d in the gel matrix; System DiaMed neutral: DiaMed ID NaCl, enzyme test and cold agglutinins micro column test card, containing no free antibodies in the gel matrix.

The invention will now be further explained in and by the following examples. It is of importance to see, however, that these examples are given for illustrative purposes and illuminate the enablement of the invention but do not in any way constitute the definitive conditions of the different assay methods nor limit the scope of the invention in any way.

### Examples

### Example 1

10 ml Sepharose HP (GE Healthcare/Amersham, Uppsala, Sweden) was washed according to the instructions of the supplier and included in a 20 mM phosphate buffer, further containing 70 mM NaCl, 20 mM EDTA, 30 mM glucose, 2.5% dextran, 2.0% BSA, pH 8.8 (gel buffer).

To this was added, polyclonal goat serum, directed to human IgG (diluted 100-fold in the final gel suspension), monoclonal mouse IgG, directed to human IgM (final concentration of 1 pg/ml) and monoclonal mouse IgG, directed to human C3d (final concentration of 5 pg/ml).

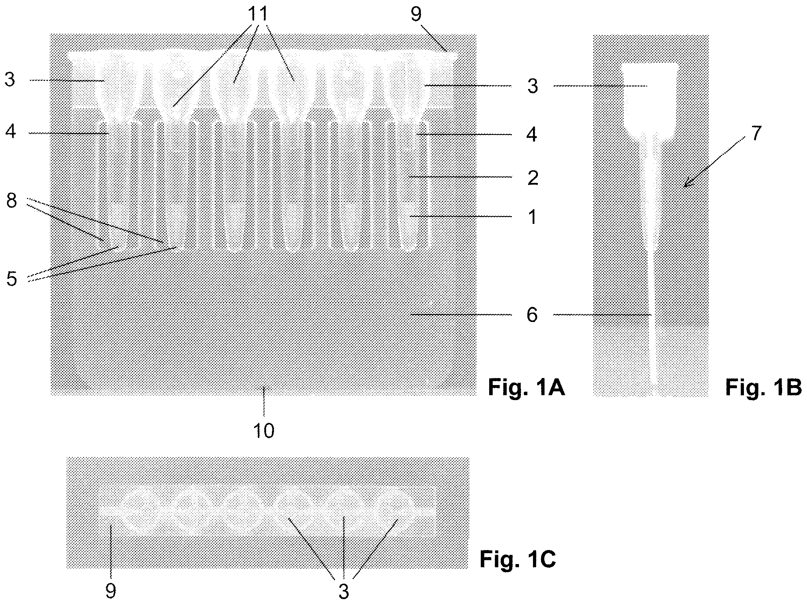

Additional gel buffer was added until a final gel suspension of 23% was obtained. Per micro column of a test card according to **Figure 1****,** 22 µl gel suspension was added. The gel sedimented within 15 min (yielding about 5 µl of packed gel, i.e., the present third elongated compartment). The supernatant gel buffer (about 17 µl) functions as a layer of high density separation medium, i.e., the present second elongated compartment.

Red blood cells of blood group 0+ (i.e. blood group 0, Rhesus D positive) were sensitized with IgG type anti-D antibodies and then 3 times washed in a modified LISS (low ionic strength solution). Of these red blood cells, a 0.5% suspension was made in LISS. Then, 1 drop of this suspension was introduced into the incubation compartment of the micro column. To this was added, 1 drop of normal serum from a donor of blood group 0+.

After an incubation period of 15 min at 37°C, the micro column was centrifuged using the following protocol: 2 min at 75xg, followed by 1 min at 200xg, followed by 7 min at 1790xg.

Then the result was assessed: all red blood cells were agglutinated on top of the inert gel matrix (reaction strength 4+). As a control experiment, the procedure was repeated with non-sensitized red cells of blood group 0+. These cells were not agglutinated but collected at the bottom of the micro column.

### Example 2

A micro column card as described in Example 1 was prepared. Of Duffy-a (Fya) positive red blood cells (phenotype Fya+b+), a 0.5% suspension in LISS was made. Then 1 drop of this suspension was introduced into the incubation compartment of the micro column. To this was added 1 drop of anti-Fya (polyclonal IgG antiserum, Sanquin Reagents, Amsterdam, Netherlands).

After an incubation period of 15 min at 37°C, the micro column was centrifuged according to the protocol of Example 1.

After this, the result was assessed: all red cells were agglutinated on top of the inert gel matrix (reaction strength 4+). As a control experiment, corresponding non-sensitized red cells were tested. These cells were not agglutinated but collected at the bottom of the micro column.

This experiment was repeated for the following antigens with the corresponding polyclonal IgG antisera: Fyb (with anti-Fyb serum and a Fya+b+ cell), Jka (with anti-Jka serum and a Jka+b+ cell), Jkb (with anti-Jkb serum and a Jka+b+ cell), Kell (with anti-K serum and a K+k+ cell), cellano (with anti-k serum and a K+k+ cell), Lua (with anti-Lua serum and a Lua+b+ cell), Lub (with anti-Lub serum and a Lua+b+ cell), S (with anti-S serum and a S+s+ cell) and s (with anti-s serum and a S+s+ cell). The results were similar to the results described for Fya.

In all cases, complete agglutination of red cells on top of the inert gel matrix occurred (reaction strength 4+), while non-sensitized red cells in the control experiments were not agglutinated but collected at the bottom of the micro column.

### Example 3

A micro column card as described in Example 1 was prepared. Of red blood cells carrying the A antigen (A positive cells), a 0.5% suspension in LISS was made. Then 1 drop of this suspension was introduced into the incubation compartment of the micro column. To this was added 1 drop of anti-A (monoclonal IgM reagent, Sanquin Reagents, Amsterdam, Netherlands). Next, the micro column was centrifuged according to the protocol of Example 1.

After this, the result was assessed: all red cells were agglutinated on top of the inert gel matrix (reaction strength 4+). As a control experiment, corresponding non-sensitized red cells were tested. These cells were not agglutinated but collected at the bottom of the micro column.

This experiment was repeated for the following antigens with the corresponding monoclonal IgM reagents: B (with anti-B and a B positive cell), D (with anti-D and a D positive cell), C (with anti-C and a C+c+ cell), c (with anti-c and a C+c+ cell), E (with anti-E and a E+e+ cell), e (with anti-e and a E+e+ cell) and Kell (with anti-K and a K+k+ cell). The results were in agreement with what has been described for the A positive cell. In all cases, complete agglutination of red cells on top of the inert gel matrix occurred, while non-sensitized red cells in the control experiments were not agglutinated but collected at the bottom of the micro column.

### Example 4

In this example, the following test systems were assayed:

### System 1:

The test system as described in Example 1 having free anti-human IgG, anti-human IgM and anti-human C3d in the high density medium.

### System 2:

As system 1, but with free anti-human IgG alone in the high density medium.

### System 3:

As system 1, but without free antibodies in the high density medium.

### System 4:

As system 1, but without the layer of high density medium above of the gel matrix. To that end the layer of high density medium was carefully removed with a capillary tip, leaving the gel particles settled and just covered with liquid medium, still containing the potentiators and free antibodies.

### System DiaMed LISS/Coombs:

DiaMed ID LISS/Coombs micro column test card, containing free anti-human IgG and anti-human C3d in the gel matrix.

### System DiaMed neutral:

DiaMed ID NaCl, enzyme test and cold agglutinins micro column test card, containing no free antibodies in the gel matrix.

The last 2 systems were used according to the manufacturer's instructions.

Some of the above-mentioned test systems were examined for their ability to demonstrate red cells loaded with complement-components in vitro (C3b cells, C3d cells) or in vivo (patient samples). For this purpose fresh citrate-blood and an MgCl₂-solution were added to a cold sucrose-solution.

After 45 min of slowly stirring the mixture, the resulting C3b cells were washed 4 times with cold PBS. A part of the C3b cells was treated with trypsin after which the resulting C3d cells were washed 3 times with an excess of PBS.

In addition, two patient samples were used, which were known to be loaded in vivo with complement components only. Of these C3b, C3d and patient cells, respectively, a 0.5% suspension in LISS was prepared. Then 1 drop of this suspension was introduced into the incubation compartment of the micro column. Directly hereafter the micro columns were centrifuged according to the protocol of Example 1.

After this, the result was assessed. The reaction strengths are summarized in Table 1. As a control, experiments were carried out with untreated cells instead of cells loaded with complement-components, yielding only negative reactions (results not shown).

**Table 1**

| **Sample** | **System 1¹** | **System 2** | **System 3** |
|---|---|---|---|
| C3b cells | 4+ | - | - |
| C3d cells | 4+ | - | - |
| Patient 1 | 2+ | - | - |
| Patient 2 | 2+ | - | - |

| | | | |
|---|---|---|---|
| ¹⁾ 4+: all agglutinated cells on top of the inert gel matrix, no cells at the bottom; 3+more agglutinated cells on top of the inert gel matrix than non-agglutinated cells at the bottom; 2+: as many agglutinated cells on top of the inert gel matrix as non-agglutinated cells at the bottom; 1+: fewer agglutinated cells on top of the inert gel matrix than non-agglutinated cells at the bottom; (+): very few agglutinated cells on top of the inert gel matrix, many non-agglutinated cells at the bottom; -: no agglutinated cells on top of the inert gel matrix, all cells at the bottom | | | |

### Example 5

Several of the above-mentioned test systems were examined for their ability to demonstrate complement-dependent antibodies. For this, two patient sera were tested which were known to contain complement-dependent IgG type anti-Jka and anti-Jkb antibodies, respectively. Of Jka+b-cells and Jka-b+ cells, respectively, a 0.5% suspension in LISS was prepared. Then 1 drop of this suspension was introduced into the incubation compartment of the micro column. To this was added 1 drop of patient serum.

After an incubation period of 15 min at 37°C, the micro columns were centrifuged according to the protocol of Example 1. After this, the result was assessed. The reaction strengths are summarized in **Figure 3****.** As a control, experiments were carried out with AB-serum instead of patient serum, yielding only negative reactions (results not shown) .

As can be seen in **Figure 3****,** systems 1 and 2 provide a clear (positive) read out shown at two levels. This in contrast with the system according to the prior art showing a diffuse smear over at least part of the column length (upper part of **Figure 3**), or a false negative reaction (lower part **Figure 3**).

### Example 6

Several of the above-mentioned test systems were examined for their ability to demonstrate (weak) IgG type antibodies. For this purpose, two patient sera were tested which were known to contain (weak) IgG type antibodies (anti-K and anti-Fya). Of K+k+ cells and of Fya+b+ cells, respectively, a 0.5% suspension in LISS was prepared. Then 1 drop of red cell suspension was introduced into the incubation compartment of the micro column. To this was added 1 drop of patient serum.

After an incubation period of 15 min at 37°C, the micro columns were centrifuged according to the protocol of Example 1. After this, the result was assessed. The reaction strengths are summarized in **Figure 4****.** As a control, experiments were carried out with AB-serum instead of patient serum, yielding only negative reactions (results not shown) .

As can be seen in **Figure 4****,** systems 1 and 2 according to the present invention provide a clear (positive) read out shown at two levels. This in contrast with the system according to the prior art showing a diffuse smear over at least part of the column length.

### Example 7

All of the above-mentioned test systems were examined for their ability to demonstrate weak IgM type antibodies. For this purpose, two patient plasma samples were tested which were known to contain weak IgM type antibodies (anti-Lea (IgM) and anti-s (IgM + IgG)). Of Lea+b+ cells and S+s+ cells, respectively, a 0.5% suspension in LISS was prepared. Then 1 drop of red cell suspension was introduced into the incubation compartment of the micro column. To this was added 1 drop of patient plasma. After an incubation period of 15 min at 37°C, the micro columns were centrifuged according to the protocol of Example 1.

After this, the result was assessed. The reaction strengths are summarized in **Figure 5****.** As a control, experiments were carried out with AB-plasma instead of patient serum, yielding only negative reactions (results not shown).

As can be seen in **Figure 5****,** systems 1 and 2 according to the present invention provide a clear (positive) read out shown at two levels. This in contrast with the systems according to the prior art maximally showing a diffuse smear over at least part of the column length or a false negative reaction.

### Example 8

Similar to example 7, six weak AB0-incompatibilities were tested, viz. A2B red cell samples with blood group B plasma samples (containing IgM type anti-A antibodies), which in a normal tube agglutination test in a physiological salt solution still gave a 1+ reaction.

Of the different A2B red cells, a 0.5% suspension in LISS was prepared. Then 1 drop of red cell suspension was introduced into the incubation compartment of the micro column. To this was added 1 drop of patient plasma. After an incubation period of 15 min at 37°C, the micro columns were centrifuged according to the protocol of Example 1.

After this, the result was assessed. The reaction strengths are summarized in **Figure 6****.** As a control, experiments were carried out with AB-plasma instead of group B plasma, yielding only negative reactions (results not shown) .

As can be seen in **Figure 6****,** system 1 according to the present invention provides a clear (positive) read out shown at two levels. This in contrast with the systems according to the prior art maximally only showing false negative reactions.

### Conclusions

From **Figure 3** it is clear that complement dependent IgG antibodies are demonstrated with a higher sensitivity according to the present invention (systems 1 and 2). The addition of, next to free anti-human IgG, free anti-human C3d as a ligand in the high density medium of the test system (system 1) further increased the sensitivity.

From **Figure 5** and **Figure 6****,** it is clear that weak agglutinins reactive at 37 °C are demonstrated with a higher sensitivity according to the present invention. When, next to free anti-human IgG and free anti-human C3d, also free anti-human IgM is present as a ligand in the high density medium of the test system the sensitivity is further increased (system 1). Moreover, in the absence of free anti-human IgM some weak agglutinins reactive at 37 °C can no longer be demonstrated (system 2 and system 3).

From **Figures 3-6****,** it is clear that the presence of the second compartment according to the present invention is essential for obtaining a correct test result. Without this (system 4) red blood cell agglutination still seems feasible to some extent, due to the presence of free ligands in the medium between the gel beads.

The results, however, show a (vague) smear of red cells instead of a sharp and distinct red line on top of the gel matrix, thereby excluding the possibility of reading the result on only two levels (underneath and on top of the gel matrix). As a consequence the reaction strength can no longer be interpreted.

Moreover, some weak IgG type antibodies and weak agglutinins reactive at 37 °C could no longer be demonstrated when the high density medium above the gel matrix was removed (system 4).

## Claims

1. Micro column (7) capable of detecting agglutinated-antibody-loaded-erythrocytes comprising at least three fluid communicating elongated compartments (1, 2 and 3), wherein said micro column (7) comprises:
a) a first elongated compartment (3) capable of receiving erythrocytes and/or antibodies, thereby providing a sample with a first liquid density, for forming antibody-loaded-erythrocytes, but not agglutinated-antibody-loaded-erythrocytes, wherein said first compartment (3) is in liquid communication with;
b) a second elongated compartment (2) of about 17 µl consisting of a medium with a second liquid density, wherein said second fluid density is higher than said first fluid density, wherein said medium comprises at least one compound selected from the group consisting of anti-IgG antibodies, anti-IgM antibodies, anti-C3d antibodies, anti-IgA antibodies, anti-C3c antibodies, and functional fragments thereof and wherein said second liquid density is between 1.01 to 1.09 g/ml, preferably between 1.02 and 1.06 g/ml, more preferably between 1.02 and 1.04 g/ml, for forming agglutinated-antibody-loaded-erythrocytes, and wherein said second compartment (2) is in liquid communication with;
c) a third elongated compartment (1) of about 5 µl consisting of inert beads, comprised in the medium according to (b), capable of providing a barrier for said agglutinated-antibody-loaded erythrocytes and not for said antibody-loaded-erythrocytes, erythrocytes and/or antibodies;
wherein the ratio of the length of said elongated second compartment (2) to said third elongated compartment (1) is between 1.1 to 1.7 thereby providing complex formation and agglutination of said antibody-loaded-erythrocytes at the interface of said second (2) and third (1) elongated compartment.

2. Micro column (7) according to claim 1, wherein the top-end diameter of said first elongated compartment (3) is larger than the bottom-end diameter of said first elongated compartment (3).

3. Micro column (7) according to any of the claims 1 to 2, wherein said medium with a second liquid density comprises 2% to 4% of dextran and 1% to 3% serum albumin with a pH of at least 6.

4. Micro column (7) according to claim 3, wherein said medium with a second liquid density further comprises a physiological salt solution, a chelator and a mono- or disaccharide.

5. Micro column (7) according to claim 3 or claim 4, wherein said medium with a second liquid density further comprises 15 to 25 mM EDTA, 60 to 80 mM NaCl, 20 to 40 mM glucose in a phosphate buffered solution (PBS).

6. Micro column (7) according to any of the claims 3 to 5, wherein said medium with a second liquid density comprises 2.5% dextran, 2% serum albumin, 20 mM EDTA, 70 mM NaCl, and 30 mM glucose in a phosphate buffered solution (PBS).

7. Micro column (7) according to claim 1 comprising anti-IgG antibodies, anti-IgM antibodies, anti-C3d antibodies.

8. Micro column (7) according to any of the claims 1 to 7, wherein said inert beads, comprised in the medium according to (b), have an average diameter of between 10 to 150 µm, preferably between 20 to 50 µm.

9. Micro column (7) according to any of the claims 1 to 8, wherein said inert beads, comprised in the medium according to (b), are selected from the group consisting of glass, sepharose, acryl, crosslinked agarose, and sephadex beads.

10. Method for detecting agglutinated antibody-loaded-erythrocytes comprising:
a) loading erythrocytes and antibodies into a first elongated compartment (3) of a micro column (7) as defined in any of the claims 1 to 9;
b) incubating said antibodies and said erythrocytes in said first elongated first compartment (3) for forming antibody-loaded-erythrocytes;
c) introducing, by centrifugation, said antibody-loaded-erythrocytes into a second elongated compartment (2) of a Micro column (7) as defined in any of the claims 1 to 9 for forming agglutinated-antibody-loaded-erythrocytes;
d) separating, by centrifugation, said agglutinated-antibody-loaded-erythrocytes from said antibody-loaded-erythrocytes and said erythrocytes in a third elongated compartment (1) of a Micro column (7) as defined in any of the claims 1 to 9;
e) detecting the presence of agglutinated-antibody-loaded-erythrocytes at the interface of said second (2) and third (1) elongated compartment of a micro column (7) as defined in any of the claims 1 to 9.

11. Method according to claim 10, wherein the incubation comprises 1 second to 30 minutes at 15 to 37 °C.

12. Method according to claim 10 or claim 11, wherein the centrifugation as defined in steps (c) and (d) comprises 1 to 3 minutes at 50 to 100xg; 0.5 to 1.5 minutes at 150 to 250xg; and 5-10 minutes at 1500 to 2000xg, preferably wherein the centrifugation as defined in steps (c) and (d) comprises 2 minutes at 75xg; 1 minute at 200xg; and 7 minutes at 1790xg.

13. Kit of parts for carrying out a method as defined in any of the claims 10 to 12 comprising:
a) at least one micro column (7) as defined in any of the claims 1 to 9;
b) instructions for use; and
c) optionally, one or more control analytes and/ or carriers.

## Patentansprüche

1. Mikrosäule (7), geeignet, agglutinierter-Antikörper-beladene Erythrozyten zu detektieren, umfassend mindestens drei flüssig kommunizierende, längliche Kompartimente (1, 2 und 3), wobei die Mikrosäule (7) umfasst:
a) ein erstes längliches Kompartiment (3), geeignet, um Erythrozyten und/oder Antikörper aufzunehmen, um auf diese Weise eine Probe mit einer ersten Flüssigkeitsdichte bereitzustellen, für das Ausbilden Antikörper-beladener Erythrozyten, aber nicht agglutinierter-Antikörper-beladener Erythrozyten, wobei das erste Kompartiment (3) in Flüssigkeitsverbindung steht mit;
b) einem zweiten länglichen Kompartiment (2) von ungefähr 17 µl bestehend aus einem Medium mit einer zweiten Flüssigkeitsdichte, wobei die zweite Flüssigkeitsdichte höher ist als die erste Flüssigkeitsdichte, wobei das Medium mindestens eine Verbindung ausgewählt aus der Gruppe bestehend aus anti-IgG-Antikörpern, anti-IgM-Antikörpern, anti-C3d-Antikörpern, anti-IgA-Antikörpern, anti-C3c-Antikörpern, und funktionalen Fragmenten davon umfasst und wobei die zweite Flüssigkeitsdichte zwischen 1,01 bis 1,09 g/ml, vorzugsweise zwischen 1,02 und 1,06 g/ml, noch bevorzugter zwischen 1,02 und 1,04 g/ml beträgt, für das Ausbilden agglutinierter-Antikörper-beladener Erythrozyten, und wobei das zweite Kompartiment (2) in Flüssigkeitsverbindung steht mit;
c) einem dritten länglichen Kompartiment (1) von ungefähr 5 µl bestehend aus inerten Kügelchen, umfasst in dem Medium gemäß (b), geeignet, um eine Barriere für die agglutinierter-Antikörper-beladenen Erythrozyten und nicht für die Antikörper-beladenen Erythrozyten, Erythrozyten und/oder Antikörper bereitzustellen;
wobei das Längenverhältnis des länglichen zweiten Kompartiments (2) zu dem dritten länglichen Kompartiment (1) zwischen 1,1 bis 1,7 ist, wodurch Komplexbildung und Agglutination der Antikörper-beladenen Erythrozyten an der Grenzfläche des zweiten (2) und dritten (1) länglichen Kompartiments bereitgestellt wird.

2. Mikrosäule (7) gemäß Anspruch 1, wobei der Durchmesser des oberen Endes des ersten länglichen Kompartiments (3) größer ist als der Durchmesser des unteren Endes des ersten länglichen Kompartiments (3).

3. Mikrosäule (7) gemäß einem der Ansprüche 1 bis 2, wobei das Medium mit einer zweiten Flüssigkeitsdichte 2 % bis 4 % Dextran und 1 % bis 3 % Serumalbumin mit einem pH von mindestens 6 umfasst.

4. Mikrosäule (7) gemäß Anspruch 3, wobei das Medium mit einer zweiten Flüssigkeitsdichte des Weiteren eine physiologische Salzlösung, einen Chelatbildner und ein Mono- oder Disaccharid umfasst.

5. Mikrosäule (7) gemäß Anspruch 3 oder Anspruch 4, wobei das Medium mit einer zweiten Flüssigkeitsdichte des Weiteren 15 bis 25 mM EDTA, 60 bis 80 mM NaCl, 20 bis 40 mM Glucose in einer Phosphat-gepufferten Lösung (PBS) umfasst.

6. Mikrosäule (7) gemäß einem der Ansprüche 3 bis 5, wobei das Medium mit einer zweiten Flüssigkeitsdichte 2,5 % Dextran, 2 % Serumalbumin, 20 mM EDTA, 70 mM NaCl und 30 mM Glucose in einer Phosphat-gepufferten Lösung (PBS) umfasst.

7. Mikrosäule (7) gemäß Anspruch 1 umfassend anti-IgG Antikörper, anti-IgM-Antikörper, anti-C3d-Antikörper.

8. Mikrosäule (7) gemäß einem der Ansprüche 1 bis 7, wobei die inerten Kügelchen, umfasst in dem Medium gemäß (b), einen durchschnittlichen Durchmesser von zwischen 10 bis 150 µm, vorzugsweise zwischen 20 bis 50 µm haben.

9. Mikrosäule (7) gemäß einem der Ansprüche 1 bis 8, wobei die inerten Kügelchen, umfasst in dem Medium gemäß (b), ausgewählt sind aus der Gruppe bestehend aus Glas-, Sepharose-, Acryl-, vernetzte-Agarose-, und Sephadex-Kügelchen.

10. Verfahren zum Detektieren agglutinierter-Antikörper-beladener Erythrozyten, umfassend:
a) Laden von Erythrozyten und Antikörpern in ein erstes längliches Kompartiment (3) einer Mikrosäule (7) wie in einem der Ansprüche 1 bis 9 definiert;
b) Inkubieren der Antikörper und der Erythrozyten in dem ersten länglichen Kompartiment (3), um Antikörper-beladene Erythrozyten auszubilden;
c) Einführen, mittels Zentrifugation, der Antikörper-beladenden Erythrozyten in ein zweites längliches Kompartiment (2) einer Mikrosäule (7) wie in einem der Ansprüche 1 bis 9 definiert, um agglutinierter-Antikörper-beladene Erythrozyten auszubilden;
d) Separieren, mittels Zentrifugation, der agglutinierter-Antikörper-beladende Erythrozyten von den Antikörper-beladenen Erythrozyten und den Erythrozyten in einem dritten länglichen Kompartiment (1) einer Mikrosäule (7) wie in einem der Ansprüche 1 bis 9 definiert;
e) Nachweisen der Anwesenheit von agglutinierter-Antikörper-beladenen Erythrozyten an der Grenzfläche des zweiten (2) und des dritten (1) länglichen Kompartiments einer Mikrosäule (7) wie in einem der Ansprüche 1 bis 9 definiert.

11. Verfahren gemäß Anspruch 10, wobei das Inkubieren 1 Sekunde bis 30 Minuten bei 15 bis 37 °C umfasst.

12. Verfahren gemäß Anspruch 10 oder Anspruch 11, wobei die Zentrifugation wie in den Schritten (c) und (d) definiert 1 bis 3 Minuten bei 50 bis 100 xg; 0,5 bis 1,5 Minuten bei 150 bis 250 xg; und 5-10 Minuten bei 1500 bis 2500 xg umfasst, vorzugsweise wobei die Zentrifugation wie in den Schritten (c) und (d) definiert 2 Minuten bei 75 xg; 1 Minute bei 200 xg; und 7 Minuten bei 1790 xg umfasst.

13. Teilesatz zum Durchführen eines Verfahrens wie in einem der Ansprüche 10 bis 12 definiert, umfassend:
a) mindestens eine Mikrosäule (7) wie in einem der Ansprüche 1 bis 9 definiert;
b) Gebrauchsanweisungen; und
c) wahlweise, einen oder mehrere Kontrollanalyten und/oder Träger.

## Revendications

1. Micro-colonne (7) apte à détecter des agglutinats d'érythrocytes chargés d'anticorps comprenant au moins trois compartiments allongés en communication fluidique (1, 2 et 3), dans laquelle ladite micro-colonne (7) comprend :
a) un premier compartiment allongé (3) apte à recevoir des érythrocytes et/ou des anticorps, fournissant ainsi un échantillon ayant une première masse volumique de liquide, pour former des érythrocytes chargés d'anticorps, mais pas d'agglutinats d'érythrocytes chargés d'anticorps, dans laquelle ledit premier compartiment (3) est en communication liquide avec ;
b) un deuxième compartiment allongé (2) d'environ 17 µl constitué d'un milieu ayant une seconde masse volumique de liquide, dans laquelle ladite seconde masse volumique de liquide est supérieure à ladite première masse volumique de liquide, dans laquelle ledit milieu comprend au moins un composé choisi dans le groupe constitué d'anticorps anti-IgG, d'anticorps anti-IgM, d'anticorps anti-C3d, d'anticorps anti-IgA, d'anticorps anti-C3c, et de fragments fonctionnels de ceux-ci et dans laquelle ladite seconde masse volumique de liquide est entre 1,01 et 1,09 g/ml, de préférence entre 1,02 et 1,06 g/ml, de manière davantage préférée entre 1,02 et 1,04 g/ml, pour former des agglutinats d'érythrocytes chargés d'anticorps, et dans laquelle ledit deuxième compartiment (2) est en communication liquide avec ;
c) un troisième compartiment allongé (1) d'environ 5 µl constitué de billes inertes, comprises dans le milieu selon (b), apte à fournir une barrière pour lesdits agglutinats d'érythrocytes chargés d'anticorps et pas pour lesdits érythrocytes chargés d'anticorps, érythrocytes et/ou anticorps ;
dans laquelle le rapport de la longueur dudit deuxième compartiment allongé (2) audit troisième compartiment allongé (1) est entre 1,1 et 1,7 fournissant ainsi une formation de complexes et une agglutination desdits érythrocytes chargés d'anticorps au niveau de l'interface dudit deuxième (2) et dudit troisième (1) compartiment allongé.

2. Micro-colonne (7) selon la revendication 1, dans laquelle le diamètre de l'extrémité supérieure dudit premier compartiment allongé (3) est plus grand que le diamètre de l'extrémité inférieure dudit premier compartiment allongé (3).

3. Micro-colonne (7) selon l'une quelconque des revendications 1 à 2, dans laquelle ledit milieu ayant une seconde masse volumique de liquide comprend de 2 % à 4 % de dextrane et de 1 % à 3 % de sérumalbumine à un pH d'au moins 6.

4. Micro-colonne (7) selon la revendication 3, dans laquelle ledit milieu ayant une seconde masse volumique de liquide comprend en outre une solution saline physiologique, un chélateur et un mono- ou disaccharide.

5. Micro-colonne (7) selon la revendication 3 ou la revendication 4, dans laquelle ledit milieu ayant une seconde masse volumique de liquide comprend en outre 15 à 25 mM d'EDTA, 60 à 80 mM de NaCl, 20 à 40 mM de glucose dans une solution tamponnée au phosphate (PBS).

6. Micro-colonne (7) selon l'une quelconque des revendications 3 à 5, dans laquelle ledit milieu ayant une seconde masse volumique de liquide comprend 2,5 % de dextrane, 2 % de sérumalbumine, 20 mM d'EDTA, 70 mM de NaCl et 30 mM de glucose dans une solution tamponnée au phosphate (PBS).

7. Micro-colonne (7) selon la revendication 1 comprenant des anticorps anti-IgG, des anticorps anti-IgM, des anticorps anti-C3d.

8. Micro-colonne (7) selon l'une quelconque des revendications 1 à 7, dans laquelle lesdites billes inertes, comprises dans le milieu selon (b), ont un diamètre moyen entre 10 et 150 µm, de préférence entre 20 et 50 µm.

9. Micro-colonne (7) selon l'une quelconque des revendications 1 à 8, dans laquelle lesdites billes inertes, comprises dans le milieu selon (b), sont choisies dans le groupe constitué de billes de verre, de sépharose, d'acryle, d'agarose réticulé et de Sephadex.

10. Procédé de détection d'agglutinats d'érythrocytes chargés d'anticorps comprenant :
a) le chargement d'érythrocytes et d'anticorps dans un premier compartiment allongé (3) d'une micro-colonne (7) selon l'une quelconque des revendications 1 à 9 ;
b) l'incubation desdits anticorps et desdits érythrocytes dans ledit premier compartiment allongé (3) pour former des érythrocytes chargés d'anticorps ;
c) l'introduction, par centrifugation, desdits érythrocytes chargés d'anticorps dans un deuxième compartiment allongé (2) d'une micro-colonne (7) selon l'une quelconque des revendications 1 à 9 pour former des agglutinats d'érythrocytes chargés d'anticorps ;
d) la séparation, par centrifugation, desdits agglutinats d'érythrocytes chargés d'anticorps desdits érythrocytes chargés d'anticorps et desdits érythrocytes dans un troisième compartiment allongé (1) d'une micro-colonne (7) selon l'une quelconque des revendications 1 à 9 ;
e) la détection de la présence d'agglutinats d'érythrocytes chargés d'anticorps au niveau de l'interface dudit deuxième (2) et dudit troisième (1) compartiment allongé d'une micro-colonne (7) selon l'une quelconque des revendications 1 à 9.

11. Procédé selon la revendication 10, dans lequel l'incubation comprend 1 seconde à 30 minutes de 15 à 37 °C.

12. Procédé selon la revendication 10 ou la revendication 11, dans lequel la centrifugation telle que définie dans les étapes (c) et (d) comprend 1 à 3 minutes de 50 à 100xg ; 0,5 à 1,5 minute de 150 à 250xg ; et 5 à 10 minutes de 1 500 à 2 000xg, de préférence dans lequel la centrifugation telle que définie dans les étapes (c) et (d) comprend 2 minutes à 75xg ; 1 minute à 200xg ; et 7 minutes à 1790xg.

13. Kit d'éléments pour réaliser un procédé selon l'une quelconque des revendications 10 à 12 comprenant :
a) au moins une micro-colonne (7) selon l'une quelconque des revendications 1 à 9 ;
b) des instructions d'utilisation ; et
c) facultativement, un ou plusieurs analytes témoins et/ou supports.
